# EUROPEAN PATENT SPECIFICATION

(11) **EP 3 941 397 B1**
(45) Date of publication and mention of the grant of the patent: **03.09.2025**
(21) Application number: 20714496.5
(22) Date of filing: 17.03.2020
(51) Int. Cl.: A61F 2/24, A61B 17/29, A61B 17/06

(54) **INSTRUMENT FOR REPAIRING AN ATRIOVENTRICULAR HEART VALVE**
INSTRUMENT ZUR REPARATUR EINER ATRIOVENTRIKULÄREN HERZKLAPPE
INSTRUMENT POUR RÉPARER UNE VALVULE CARDIAQUE ATRIO-VENTRICULAIRE

(30) Priority: 19.03.2019 CH 3532019
(43) Date of publication of application: 26.01.2022
(73) Proprietor: Coremedic GmbH, 78315 Radolfzell am Bodensee (DE)
(72) Inventor: BAUER, Thomas, 78476 Allensbach (DE); SIRONI, Tommaso, 78467 Konstanz (DE); ROTH, Michael, 78315 Radolfzell (DE); SCHAFFNER, Silvio, 8267 Berlingen (CH); GUIDOTTI, Andrea, 8702 Zollikon (CH); ZARBATANY, David, Laguna Niguel, CA 92677 (US); FLORES, Jesus, Mission Viejo, California 92691 (US); STEFOPOULOS, Georgios, 8050 Zürich (CH); VICENTINI, Luca, 8057 Zürich (CH); ARDUINI, Mattia, 01170 Chevry (FR)
(74) Representative: Frei Patent Attorneys
(86) International application number: PCT/EP2020/057350
(87) International publication number: WO 2020/187944

(56) References cited:
- WO-A1-2017/066890
- WO-A1-2017/066890
- WO-A2-2006/078694
- WO-A2-2006/078694
- WO-A2-2008/112237
- WO-A2-2008/112237
- WO-A2-2012/006161
- WO-A2-2012/006161
- US-A1- 2004 044 365
- US-A1- 2004 044 365
- US-A1- 2006 271 073
- US-A1- 2006 271 073
- US-A1- 2007 049 952
- US-A1- 2007 049 952
- US-A1- 2009 105 729
- US-A1- 2009 105 729
- US-A1- 2009 299 406
- US-A1- 2009 299 406
- US-A1- 2012 184 971
- US-A1- 2012 184 971
- US-A1- 2016 220 372
- US-A1- 2016 220 372

## Description

### FIELD OF THE INVENTION

The invention is in the field of minimally invasive surgical and interventional cardiology devices for heart valve repair. It particularly relates to an instrument for repairing an atrioventricular heart valve, mitral heart valve or also the tricuspid heart valve in a minimally invasive manner.

### BACKGROUND OF THE INVENTION

Prolapses of a leaflet of the mitral valve into the left atrium and resulting valve insufficiency can cause serious dysfunctions of the heart. One reason for such prolapse are damaged tendons (chordae tendineae) that connect the leaflets of the mitral valve to the papillary muscle through the left ventricle. Such damage may for example be a result of a myocardial infarction, tissue degeneration or infectious disease.

A repair of such a prolapse demands the leaflet or leaflets to be re-connected to the papillary muscle, for example by synthetic fibers, such as Gore-Tex^{®} fibers. Such an approach in accordance with the state of the art demands suturing the implant to a papillary muscle. A first disadvantage of such a repair process is that it is only possible while the heart is inactive, thus the surgical repair demands that the heart is stopped and drained of blood, while a cardiopulmonary bypass is used. A second disadvantage is that the success of the operation depends strongly on the skill of the surgeon. A further disadvantage is that the fibers sutured to the leaflet may cause long-time damage.

Most laparoscopic and minimally invasive techniques and tools that have been developed are not usable for the requirements of mitral valve repair on a beating heart. While certain annuloplasty techniques and instruments that can suture an annuloplasty ring as part of vascular repair or heart bypass surgery may be used in conjunction with a beating heart, these annuloplasty procedures.

WO 2009/052528 A2 refers to a device for performing minimally invasive valve leaflet repair in a beating heart performing a clamping mechanism adapted to grasp and release the valve leaflet. Therefore the instrument includes a needle assembly and a suture cartridge. Before starting the operation a suture to be used has to be threaded through openings on the cartridge assembly. The mechanism performed by that instrument includes drawing a suture through the leaflet and using a prolene suture to tension the first suture. Subsequently, each suture has to be secured on the epicardium adjacent to a ventriculotomy using a knot and pledget. The complete mechanism is time consuming and prone to error. In addition, methods that are based on sutures feature the disadvantage that suturing in minimally invasive setups is difficult, and therefore sufficiently reliable stability is often difficult to achieve.

WO 2017/066890 A2 discloses an instrument for repairing an atrioventricular heart valve and an according method, wherein the instrument implements a leaflet grabbing mechanism. The leaflet grabbing mechanism comprises a first arm with a proximally facing abutment surface and a second arm with a distally facing abutment surface, wherein the arms are movable with respect to one another so that the leaflet can be grabbed and clamped between the first and second abutment surfaces. It has been found that this structure causes problems during orientation and placement of the instrument using echocardiography. Furthermore, the control mechanism is rather complicated.

US 2007/049952 discloses a method and apparatus for repairing the heart's mitral valve by using anatomic restoration without the need to stop the heart. The method involves inserting a leaflet clamp through the heart's papillary muscle from which the leaflet has been disconnected, clamping the leaflet's free end and then puncturing the leaflet. One end of a suture is then passed through the hollow portion of the clamp, while the other end of the suture is maintained external to the heart. The clamp is then removed and the suture's two ends are fastened together with a securement ring/locking cap assembly to the heart wall exterior, thereby reconnecting the leaflet to the corresponding papillary muscle.

US 2009/0105729 discloses a valve repair device with a replaceable suture cartridge for repair of a valve leaflet in a beating heart.

WO 2008/112237 describes an apparatus for attaching a prosthetic tether between a leaflet of a patient's heart valve an another portion of the patient's heart to prevent prolapse of the leaflet. The apparatus releasable clamps the leaflet during attachment of the tether to the leaflet.

US 2004/0044365 discloses a single catheter mitral valve repair device for stabilizing a tissue portion and selectively applying a tissue fastener thereto. The device includes an extendable engagement tip with a vacuum port and a deployable fastener to engage the tissue.

While these inventions represent a significant advance over open heart techniques for heart valve repair, it would be advantageous to further improve upon these techniques.

### SUMMARY OF THE INVENTION

The invention is defined in the appended claims.

It is an object of the present invention to provide an instrument for repairing an atrioventricular heart valve, in particular the mitral heart valve or also the tricuspid heart valve, which instrument overcomes drawbacks of prior art devices and which ensures easy implantation, is suited also for transcatheter procedures and provides a reliable and well tissue-compliant repair. The transcatheter procedure is carried out in a minimally invasive manner on the beating heart, the leaflet has to be held still for the operation, and the instrument and method should provide solutions for this. Especially, it should easily be possible to check the orientation and placement of the instrument using echocardiopathy.

These objects are achieved by the invention as defined in the claims. In accordance with an aspect of the invention, an instrument implementing a leaflet grabbing mechanism by a leaflet grabbing structure is provided. The leaflet grabbing mechanism comprises a first, proximally facing abutment surface and a second, distally facing abutment surface, wherein the abutment surfaces are movable with respect to one another so that the leaflet can be grabbed and clamped between the first and second abutment surfaces. The instrument is suitable for transcatheter procedures.

The present invention refers to an instrument for repairing an atrioventricular heart valve in a minimally invasive manner, the instrument comprises a first tube having a tube axis defining an axial direction and a leaflet grabbing structure being arranged at a distal part of the first tube and contiguous to the first tube. The leaflet grabbing structure may comprise a main body and a jaw, whereby the leaflet grabbing structure is capable of clamping a leaflet of the heart valve between the first and second abutment surfaces. Thereby the jaw is movable relative to the main body, and comprises a first proximally facing abutment surface. The main body comprises a second, distally facing abutment surface. The instrument may further comprise a needle and in particular a cannulated needle forming an inner tube. The needle is releasable from the first tube and movable relative to the main body in axial directions. The main body and the jaw have a channel which is formed to allow the needle to extend there through while the leaflet is being clamped. The channel may have a position that is not central with respect to the tube axis. This position of the channel is chosen to allow the needle to puncture the leaflet relatively far away from the leaflet-edge. The instrument according to the present invention is in particular suitable for transcatheter procedures.

One embodiment refers to the instrument, wherein the jaw is movable in a straight axial direction. Thereby the axial direction is defined by the tube axis. This means that the jaw and the main body are separable by a movement of the jaw in axial direction (the jaw moves away from the main body; distal movement). Of course, the jaw and the main body may also be joined or merged by a movement of the jaw in axial direction (towards the main body, proximal movement). This can be used to grab a leaflet of the valve.

The needle of the instrument is cannulated to form a needle tube (called "inner tube" herein). Within the needle, the chord and for example parts of an implant may be arranged The chord may be pre-mounted to the implant parts, with a fixed or adjustable length. An adjustable length may for example be possible by a slidable knot. Alternatively, the chord may have to be mounted to the implant parts or one of the implant parts in a separate method step. The present invention refers to embodiments, wherein the cannulated needle of the instrument is arranged in an interior of the leaflet grabbing structure and is movable with respect to the leaflet grabbing structure in axial directions to be released from the interior. Therefore, the grabbing structure has a channel, wherein the cannulated needle may be arranged in or may be advanced through the interior of this channel. Said channel may continue at least partially the lumen of the first tube and run parallel to the tube axis through the main body as well as the jaw. The parts of the channel within the main body and the jaw can fit together so that in the closed position of the leaflet grabbing structure the lumen is continuous. The cannulated needle may be advanced through the lumen of said channel within the leaflet grabbing structure to pierce the leaflet when it is clamped between the first and second abutment surfaces.

The main body of the instrument according to the present invention may further comprise a counter portion releasable from the first tube and being movable relative to the main body in axial directions, the counter portion comprising a distally-facing second abutment surface that is pressable, by a force pointing towards a distal direction, against the first abutment surface. One embodiment refers to the instrument according to the invention, wherein the cannulated needle is arranged in an interior of the counter portion and is movable with respect to the counter portion in axial directions to be released from the interior of the counter portion to pierce the leaflet when it is clamped between the first and second abutment surfaces. The counter portion may be straight with respect to the axis and be movable in a straight axial direction.

In alternative embodiments, the grabbing structure does not have a counter portion of the main body, but the main body itself forms as the counter portion by having the distally facing second abutment surface.

In each case, the grabbing of the leaflet may be done by a relative movement of the jaw and the part (main body; separate counter portion) that has the second abutment surface. In general the main body and the jaw may be arranged to be opened and closed again. Therefore the jaw can be moved in axial direction relative to the main body, either bidirectional (back and forth) or at least unidirectional (away from the main body). In addition, a counter portion, where it exists, can be moved in axial direction, either bidirectional (back and forth) or at least unidirectional (towards the jaw). Alternatively, the main body and the jaw may be formed to be closed only. In this case the main body and the jaw are constructed to be introduced into the patient in an open form (with a certain distance) and either the jaw or the main body, respectively a part of the main body such as a counter portion, can be axially moved towards the other part.

The second abutment surface may be constituted by a press pad formed by a distal end face of a counter portion that is releasable from the first tube and/or the main body and axially movable relative to the main body. The counter portion or at least a distal end part thereof may for example be essentially tube shaped (cylindrical) or have the shape of a slitted tube to release the needle from it while the leaflet is being clamped. The counter portion may also have a rectangular, pentagonal or hexagonal cross-sectional area.

The press pad formed by the counter portion or by the main body itself may have a circular outer contour or may also be angular (such as rectangular or pentagonal or hexagonal etc.). The press pad may, especially at its distal end, be provided with a cushion to prevent the tissue from becoming damaged. The channel for the needle within the grabbing structure may be located within the main body (and, if applicable, its counter portion) and the jaw. The dimensions of the channel located in the jaw and the main body are adapted to each other so that the distally facing abutment surface and the proximally facing abutment surface of the jaw (in the leaflet grabbing position) have a substantial overlap while an axial continuation of the lumen within the counter portion coincides with the innermost portion of the channel so that a needle released from inside the counter portion while the same is pressed against the jaw is not impeded. More in particular, the mouths of the channel main body section and the channel jaw section essentially coincide when the grabbing structure is in the closed position, with the exception of a slight axial offset accounting for the thickness of the grabbed leaflet.

More in particular, the cannulated needle of the instrument may be arranged in an interior of the main body and be movable with respect to the main body in axial directions to be released from the interior of the main body (including, if applicable, the counter portion) to pierce the leaflet when it is clamped between the first and second abutment surfaces. The instrument and in particular the leaflet grabbing structure is formed in a way that the needle in a fully extended position protrude from the distal end of the leaflet grabbing structure, and in particular the distal opening of the jaw. This is important to be able to set the implant in a proper way, in particular to set the distal part of the implant in the ventricle, e.g. the papillary muscle via a transfemoral (retrograde) procedure.

In embodiments, the first and/or second abutment surfaces are structured. The structures may be selected to increase the grip of the grabbing mechanism. Therefore the structures may comprise notches, ripples, pyramids, or frustum of pyramids. It is particular suitable to structure the abutment surfaces in a way that complementary structures on both surfaces arise. Thus the first and second abutment surfaces may fit into each other.

In embodiments the first and second abutment surfaces (in the clamped position) define a plane being perpendicular to the tube axis. The abutment surfaces may also be formed to define a plane forming an angle between 20 and 90° and preferably between 30 and 80° with the tube axis. Also non-plane (curved) abutment surfaces are possible.

In embodiments, the first and second abutment surfaces encompass at least two different sections each section matching to the respective sections of the corresponding surface. The sections can differ in the structure of the surface, e.g. only one section has surface structures, such as riffles or pyramids, and the other section(s) are formed by smooth surfaces. In addition or alternatively the sections may differ in the angle formed by the tube axis and the plane defined by the respective sections of the surfaces when the surfaces abut against each other. One embodiment refers to first and second abutment surfaces formed to comprise two sections defining a plane that forms an angle with the tube axis of 90° or nearly 90° and a third section being arranged between these two sections which defines a plane forming an angle with the tube axis between 30° and 75°. This embodiment facilitates leaflet grabbing by providing a large surface area for leaflet grabbing together with an optimized grip. Therefore abutment surfaces defining a plane being oblique to the tube axis conduce to increase the area of the leaflet to be grasped. The three-sections structure of the abutment surfaces, or any other structure with abutment surfaces not perpendicular to the axis, in particular, is/are suitable to increase the distance of the area to be grasped from the edge of the leaflet.

Another embodiment of the invention refers to an instrument wherein the grabbing structure comprises at least one fold-out structure, for example a fold-out support such as wire loop or wire support to support the process of bringing the grabber into a correct orientation with respect to the leaflet and/or to increase the area of the leaflet which may be grasped and/or to stabilize the leaflet after grasping. Such a fold-out feature may be attached to the main body and/or the jaw in a way to be on the same level as the abutment surface(s). For example, a fold-out structure may be one fold-out support attached to the jaw or attached to the main body. Alternatively, the grabbing structure can comprise two fold-out supports. These can be in form of wire loops, one fixed to the jaw and one to the main body. Another possibility are two fold-out supports, both fixed either to the main body or the jaw to form one supporting structure. In case that more than one fold-out support is attached it is preferred that these wire supports are all fixed parallel in regard to the plane formed by the first and second abutment surface in closed position. Therefore it is preferred that in closed position the abutment surfaces and optionally the one or more fold-out supports define the same plane. In addition, the fold-out supports can be arranged to be folded against the grabbing structure, for example at least partially in grooves of the grabbing structure and can swing out before or during opening of the grabbing structure, for example upon being released form the outer tube.

The instrument according to the present invention may further comprise an anchor carrier arranged within the inner tube of the needle and being axially movable relative to it, the anchor carrier being configured to carry at least a part of an implant that is secured or capable of being secured to an artificial chord. In an embodiment, the implant comprises a distal implant part and a proximal implant part and a chord, wherein the chord is configured to connect and for example in the assembled state connects the proximal and distal implant parts. In a tubular element (which may be the cannulated needle or an implant sleeve inside the cannulated needle), the distal and proximal implant parts are arranged beside one another with the proximal implant part for example arranged proximally of the distal implant part. so that the tubular element prevents the proximal implant part from escaping from the anchor carrier as long as the anchor carrier is within the tubular element. Especially, the anchor carrier may axially extend within the tubular element from proximally of the proximal implant part to at least a distal end of the proximal implant part and may have a distal foot portion (stop feature) that prevents the proximal implant part from slipping out towards distally as long as it is within the tubular element. For example, the anchor carrier may comprise an anchor receiving recess (seat) proximally of the distal foot portion.

Implants of this kind are described in WO 2017/066888, and systems that comprise an anchor carrier of this kind are described in WO 2017/066889. In embodiments, instruments or sets according to the present invention may comprise an implant as described and claimed in WO 2017/066888 and/or an anchor carrier system as described and claimed in WO 2017/066889.

The grabbing structure including the main body and the jaw can be made of a metal, an alloy such as stainless steel or a polymeric material such as polyether ether ketone (PEEK). It is preferred that the grabbing structure, in particular a polymeric grabbing structure, comprises at least one marker made of a radiopaque material. This at least one marker is helpful to trace or visualize the position of the grabbing structure using X-rays. Alternatively, a radiopaque polymeric material may be used to make the grabbing structure. A polymeric material may be made radiopaque by coupling a molecule containing a dense atom (e.g. an iodine-containing molecule) onto the polymer backbone. High radiopacity of a polymeric material may alternatively be achieved by combining a polymeric resin and a powdered radiopaque agent having uniformly shaped particles of a specific particle size distribution (e.g. dense metal powders or Barium sulfate (BaSO4)).

There may also be marker which are attached to the main body and/or the jaw and which are suitable to trace the opening and closing of the grabbing structure using X-rays. The marker may be formed as strips, tapes, points or patches within the grabbing structure or attached to the grabbing structure. The marker may also be formed as small antennas or wire loops and being attached to the grabbing structure. These loops or antennas can protrude from the grabbing structure and increase the visibility using echography. Especially, they may ease determining the orientation around the axis (azimuthal position) during operation, which determination in view of the often necessarily rotationally cylindrical (rotationally cylindrical because of the transcathether approach) overall shape of the grasping device would otherwise be difficult.

Generally, markers can be made of a radiopaque polymer, an alloy, or metal, such as a platinum-iridium alloy or tantalum.

One embodiment of the grabbing structure comprises indentations or grooves wherein marker can be attached. The marker may be attached by adhesive bonding. It is preferred that at least one marker (or indentation) is located on the lateral side of the main body and at least one on the lateral side of the jaw. These markers (or indentations) are preferably located near the abutment surface or along the edge of the abutment surface and in parallel. This allows the operator (for example surgeon) to determine the orientation of the grabbing structure. Using X-ray the markers show further if the grabbing structure is closed or open and the width of the opening. A preferred form of the marker (or indentation) is curved or banana-like strip.

In embodiments, the surface of the grabbing structure or parts of the surface of the grabbing structure has a textured surface finish comprising for example convex microstructures or striated parts. Said microstructures are curved or rounded outward like the exterior of a sphere or circle. Another embodiment of the present invention refers to a grabbing structure, wherein at least a part of the surface of the grabbing structure has a roughness average Ra between 1 - 40 µm, preferably between 5 and 20 µm. Thereby surface roughness as a component of surface texture is quantified by the deviations in the direction of the normal vector of a real surface from its ideal form. The arithmetic average roughness, Ra is the arithmetic average value of filtered roughness profile determined from deviations about the center line within the evaluation length and the most widely used one-dimensional roughness parameter.

The present invention refers further to a kit (set) comprising an instrument as well as an implant suitable to be implanted by the instrument and thus, repairing an atrioventricular heart valve in particular by replacing a damaged (ruptured) chordae. Thereby the implant is preferably designed as described herein. In further embodiments, at least the proximal implant part and for example both/all implant parts may be carried by an anchor carrier which may be surrounded by the cannulated needle and possibly an additional sleeve within the cannulated needle. Therefore, a kit may comprise at least two of the following: the instrument, an anchor carrier with or without a protective sleeve, a catheter, and an implant (with or without the artificial chord). The kit can be provided comprising the elements individually or in a preassembled or assembled manner.

Especially, the proximal implant part may be assembled with the anchor carrier in a manner that it can escape and is released automatically as soon as the proximal implant part and the portion of the anchor carrier to which it is mounted is outside of the tubular element - for example without any active mechanism that causes the release, thus just by being moved out of the tubular element.

For example, the anchor carrier may axially extend within the tubular element from proximally of the proximal implant part to at least a center of the proximal implant part and for example at least to its distal end or further than its distal end. Especially, the anchor carrier may extend over the full (proximodistal) length of the proximal implant part, for example substantially as described in WO 2017/066889.

In embodiments, the anchor carrier may form a seat for the proximal implant part, out of which the proximal implant part can escape by being moved in radial direction once it is released from the tubular element, i.e. the seat is open towards one radial direction but blocks the second implant part with respect to axial directions as long as it is kept in the seat by the tubular element.

The seat for this purpose may have a structure adapted to the shape of the proximal implant part in the initial (not spread) state. Especially, the anchor carrier may have a distal foot portion with a channel for the chord, and, proximally thereof, a seat portion (also referred to as shaft portion in this text) in which the cross section is reduced to accommodate the proximal implant part. Proximally of the seat portion, the anchor carrier may have a pusher portion that has a larger cross section than the seat portion so that a pushing movement of the anchor carrier also pushes the second implant part forward as long as the second implant part is still located in the seat and not yet released.

In case the anchor carrier also carries the distal implant part in addition to the proximal implant part, it may be such that the implant parts are arranged beside one another, with the proximal implant part arranged proximally of the distal implant part. The inner tube or a sleeve element inside the inner tube may prevent the proximal implant part from escaping from the anchor carrier as long as the anchor carrier is within the inner sleeve or tube element.

The grabbing structure, as mentioned comprises a channel arranged so that the cannulated needle may be advanced through this channel so that at least part of the needle can distally protrude out of the grabbing structure. The first abutment surface may be constituted by a region around the innermost portion of the channel of the proximally facing surface of the jaw. And the second abutment surface may be constituted by a region around the innermost portion of the channel of the distally facing surface of the main body.

The channel is preferably further formed in a way that a chord, for example extending between a released distal implant part (that will be released distally of the jaw while the leaflet is being grabbed; implanted within the ventricle or a ventricle tissue) and a proximal implant part (that will be released on the atrial side of the leaflet, e.g. as anchor lying on the leaflet) can extend there through and be released by a lateral (radial) relative movement. Therefore, the channel is at least partially open to a side ("side" or "lateral" in this text unless otherwise specified are used to denote directions that are radial with respect to the tube axis). Thus, the channel extends to a lateral surface of the grabbing structure. Therefore the channel is connected to the lateral surface with a for example slit-shaped recess. Thus, the grabbing structure may comprise a slit between a lateral surface and the channel for advancing the cannulated needle. The present invention, therefore, refers to an instrument, wherein the channel is open to a single lateral side.

The opening (recess) of the channel to the single lateral side or the recess of the channel may only be located within the jaw. In this case the recess or slit runs preferably along the complete length of the jaw.

It may alternatively run through a portion of the main body and from the main body to the distal end of the jaw. Thereby the recess runs along the complete length of the jaw and a distal section of the main body. This allows that one part of the implant can be released distal of the leaflet and the other part can be released proximally of the leaflet while the leaflet is being grasped. The recess may optionally be relatively narrow, but the recess may have a wider implant release portion at the location where the proximal implant part is released while the leaflet is being clamped. The width of the recess or of its implant release portion may be between 0.4 and 0.8 mm or even extend up to a full width of the channel, and the diameter of the channel may between 1.2 and 2 mm, preferably between 1.4 and 1.8 mm.

For guiding the needle when the leaflet is pierced, especially in embodiments with a comparably wide recess (or recess implant release portion) open to a lateral side, the device may further comprise a needle guide tube encompassing the needle and being guided within the channel. Such a needle guide tube is axially movable independently of the main body and the jaw and independently of the needle at least to some extent and may be retracted relative to the needle for release of the proximal implant part.

Therefore one specific embodiment of the present invention refers to an instrument for repairing an atrioventricular heart valve in a minimally invasive manner, wherein the instrument comprises a first tube having a tube axis defining an axial direction and arranged at a distal part of the first tube and contiguous to the first tube, a leaflet grabbing structure. The leaflet grabbing structure comprises a main body and a jaw, wherein the jaw is movable relative to the main body and comprises a first proximally facing abutment surface and the main body comprises a second, distally facing abutment surface so that the leaflet grabbing structure is capable of clamping a leaflet of the heart valve between the first and second abutment surfaces. The instrument comprises further a needle wherein the needle is cannulated, forms an inner tube, and is releasable from the first tube and movable relative to the main body in axial directions. The main body and the jaw of said embodiment have a channel with a recess extending to a lateral surface wherein the recess runs through a distal portion of the main body and the complete jaw.

The main body and the jaw of the instrument may work together to form cooperatively a grasping device. Therefore, the jaw is movable relative to the main body. This movement may be produced by the operator via an operating structure, such as an operating rod. In embodiments, the operating rod is a push-pull wire. In other embodiments, the operating rod is a threaded rod. One embodiment refers to an instrument as described herein comprising an operating rod suitable to operate the axial movement of the jaw and to adjust the distance between the main body and the jaw.

Alternatively the movement may be facilitated by a cable, e.g. made of nitinol, which may be partially surrounded by a threaded sleeve, e.g. made of stainless steel. At the distal end of the rod or cable, a stop element may be attached. This stop element attaches the cable or rod to the jaw and allows axial movement of the jaw facilitated by movement of the cable or rod.

To ensure that the jaw is only moved in axial directions, the instrument may comprise at least one guiding rod (stabilizing rod) and in particular at least two stabilizing rods. The at least one guiding rod or the at least two guiding rods may be arranged to run parallel to the threaded rod (and the tube axis). The guiding rod(s) are displaceable relative to the jaw and/or the main portion to ensure a guided axial movement but in contrast to the operating structure (for example operating rod) is not equipped for being operated from outside (from the handle part).

The instrument according to the present invention is suitable for transcatheter procedures used for repairing heart valves. Therefore, the instrument according to the present invention may further comprise a guide catheter surrounding the first tube and providing a conduit to the first tube to reach the heart valve to be treated. Another additional tube of the instrument may be a steerable catheter. Steerable or deflectable catheters are known in the art of minimally invasive surgery. It would also be possible to make an interior tube steerable. Therefore, the instrument according to the present invention may further comprise or being provided as a set together with a handle and a transseptal steerable catheter.

For usage within transcatheter procedures it is advantageous if the instrument according to the present invention may have a channel for a guide wire. Thus, the present invention refers to an instrument comprising an over the wire grabbing structure. A guide wire is a device used to enter tight spaces, e.g., obstructed valves or channels, within the body, or to assist in inserting, positioning, and moving a catheter (called Seldinger Technique). Therefore the invention refers to embodiments wherein the main body and the jaw further comprise a guide wire channel suitable to incorporate a guide wire. This guide wire channel is for example located central within the grabbing structure. Alternatively, depending on the available space, the guide wire channel may be arranged off-center. The guide wire channel runs parallel to the tube axis (if it is arranged centrally coinciding therewith) from the proximal end of the main body to the distal end of jaw.

One embodiment of the present invention refers to an instrument for repairing an atrioventricular heart valve in a minimally invasive manner, the instrument comprising a first tube having a tube axis defining an axial direction and being arranged at a distal part of the first tube and contiguous to the first tube, a leaflet grabbing structure, the leaflet grabbing structure comprising a main body and a jaw, the jaw being movable relative to the main body; wherein the jaw comprises a first proximally facing abutment surface and the main body comprises a second, distally facing abutment surface; the instrument further comprising a needle wherein the needle is cannulated and forms an inner tube, and wherein the needle is releasable from the first tube and movable relative to the main body in axial directions, whereby the leaflet grabbing structure is capable of clamping a leaflet of the heart valve between the first and second abutment surfaces and wherein the grabbing structure has a channel having a position that is not central with respect to the tube axis and is formed to allow the needle to extend there through while the leaflet is being clamped. Within said embodiment the channel is open to a single lateral side. The instrument of this embodiment comprises further an operating rod suitable to operate the axial movement of the jaw and to adjust the distance between the main body and the jaw as well as at least one stabilizing rod being arranged to run parallel to the operating rod. The main body and the jaw of that embodiment may further comprise a channel suitable to incorporate a guide wire.

The cannulated needle may be made of a metal such as stainless steel. The cannulated needle may be formed to be suitable to puncture a leaflet and a ventricle tissue, e.g. a papillary muscle or the free wall of the ventricle. Therefore the cannulated needle may have a sharpened distal end. In addition it is advantageously that the cannulated needle has a certain flexibility. Therefore the cannulated needle may comprise a laser cut section proximally adjacent to the sharpened end. Therefore the distal end of the cannulated needle may be cut oblique to the tube axis. In embodiments, the cannulated needle is cut twice, with different gradients. The distal part of the cut defines a plane that forms with the tube axis an angle of 25 to 35° and the proximal part defines a plane that forms with the an angle of 15 to 22 °. Within said laser cut section the cannulated needle comprises radial laser cuts, which do not cover the complete circumference of the needle (the cuts describe an open ring).

In embodiments, the needle has a sharpened distal end and proximally therefrom a laser cut section (for flexibility). The laser cut section may be up to 30 cm long and may start 3 to 10 cm proximally of the distal end of the needle. After the laser cut section a polyimide tubing may be added having a length to reach a handle of the instrument.

A handle by which the surgeon operates the instrument may comprise a depth indicator to control the distance by which the needle has been deployed. In addition or as an alternative, the needle may comprise at least one marking that indicates the penetration depth and that may be supervised by imaging methods.

Due to the described approach, the instrument may be an all passive construction making implantation on the beating heart possible without any active power source except the pulling and pushing of wires and steering of the steerable catheter by the surgeon. Especially, it is not necessary that parts, such as an implant part, are actively shot out or similar. This makes possible a good control of the operation by the surgeon.

In embodiments, the instrument and in particular the cannulated needle of the instrument comprises the implant parts in a pre-assembled or pre-assemblable configuration. Especially, the implant parts may comprise a distal implant part to be anchored in a ventricle tissue, such as muscle tissue, and a proximal implant part to be secured to the leaflet and for example to be arranged proximally of the leaflet, with the chord extending through a perforation of the leaflet to the distal implant part.

In embodiments, the proximal implant part may be configured to lie flat on a surface of the leaflet tissue, with the chord extending from the proximal implant part through the leaflet tissue and through the ventricle to the distal implant part. To this end, the proximal implant part may for example comprise a flattish distally-facing abutment surface (distally-facing in the implanted state, i.e. facing to the side to which the chord runs). Especially, the proximal implant part may be configured to only lie on the leaflet and to thereby being secured to it - without the proximal implant part having any fastening mechanism that extends within the leaflet or through the leaflet.

The proximal implant part may hold to the leaflet without any additional fastening mechanism (such as a suture) or artificial fastening means, only by the design of the implant as such that comprises the distally facing abutment surface lying on the leaflet tissue - especially by the chord extending through the leaflet tissue and the ventricle to the distal implant part, possibly assisted by a distally-facing structure on the abutment surface that comprises portions that protrude into the tissue, without penetrating through it, and/or is indented with respect to it, to prevent shifting movements. The proximal implant part especially will, after implantation, be placed on one side of the leaflet only and not for example extend through the leaflet. The side on which the proximal implant part lies on the leaflet tissue is the atrium-facing upper side of the leaflet.

For bringing the leaflet grabbing structure from the closed position to open position or vice versa, the instrument may comprise a cable pull mechanism. Such a mechanism may for example comprise a wire appropriately guided along the first tube and the main body and the jaw, with which it is appropriately connected to pull or push it.

Of course, other variants are possible. This includes the possibility that one or more springs automatically bring the leaflet grabbing structure into the open position, and an active mechanism, such as a cable pull mechanism, is provided to bring it back into the closed position.

It is also possible that a spring mechanism is used to bring the grabbing structure into the closed position and to hold it there. Such a spring mechanism can be present everywhere in the device, for example in the handpiece held by the operator, or it may be within the outer tube that is inserted in the body. Such optional spring mechanism for closing the leaflet grabbing structure has two possible advantages: firstly, it may close the grabbing structure automatically. Secondly it exerts a controlled and constant force on the leaflet while the leaflet is being held.

In embodiments, the instrument comprises a feedback indicator showing whether or not the leaflet grabbing structure has grabbed the leaflet. Such indicator may for example comprise an optical means such as a light guide defining a light path to the distal end back, which light path is interrupted when the leaflet has been grabbed.

It has been found that the grabbing structure as described herein has a superior visibility in echography compared to devices of the prior art. Therefore, the present invention provides an instrument that provides the possibility of a good orientation using echography.

The present text also describes a method of replacing or supplementing damaged natural chordae tendineae of a human or animal heart by using a device as described herein. Features that were described in this text referring to the instrument may also belong to the method, and vice versa. Especially, this method may comprise the steps of:
- providing an instrument, the instrument comprising:
   ∘ a first tube having a tube axis defining an axial direction;
   ∘ arranged at a distal part of the first tube and contiguous to the first tube, a leaflet grabbing structure, the leaflet grabbing structure comprising a main body and a jaw, the jaw being movable relative to the main body;
   ∘ wherein the jaw comprises a first proximally facing abutment surface and the main body comprises a second, distally facing abutment surface;
   ∘ the instrument further comprising a needle wherein the needle is cannulated and forms an inner tube, and wherein the needle is releasable from the first tube and movable relative to the main body in axial directions,
   ∘ whereby the leaflet grabbing structure is capable of clamping a leaflet of the heart valve between the first and second abutment surfaces;
   ∘ and wherein the grabbing structure has a channel being formed to allow the needle to extend there through while the leaflet is being clamped and which may have a position that is not central with respect to the tube axis;
- advancing the tube from an atrial side towards a leaflet of an atrioventricular valve of the heart,
- clamping the leaflet between the first and second abutment surfaces;
- puncture the leaflet using the cannulated needle while the leaflet is being clamped;
- securing an artificial chord to the clamped leaflet; and
- removing the first tube and the leaflet grabbing structure.

The step of securing the artificial chord, may refer to a method comprising the following steps:
- providing a system arranged within the cannulated needle of the instrument, respectively the inner tube within the needle, the system comprising:
   ∘ a tubular element having an outer, distal end,
   ∘ a distal implant part arranged in the tubular element,
   ∘ the chord, being an artificial or allograft or xenograft chord arranged in the tubular element,
   ∘ a proximal implant part arranged in the tubular element, and
   ∘ an anchor carrier arranged in the tubular element,
   ∘ the distal implant part and the proximal implant part being arranged in the tubular element beside one another,
   ∘ the proximal implant part being assembled with the anchor carrier inside the tubular element so that the tubular element prevents the proximal implant part from escaping from the anchor carrier as long as the anchor carrier is within the tubular element,
- advancing the cannulated needle with the tubular element from an atrial side to a leaflet of an atrioventricular valve of the heart, piercing the leaflet and advancing the cannulated needle through the pierced leaflet and through a tissue within the ventricle, such as a papillary muscle;
- releasing tubular element from the needle and the distal implant part from the tubular element and thereby implanting the distal implant part in the tissue;
- retracting the tubular element (together with the needle) and releasing the proximal implant part proximally of the leaflet, on the atrial side thereof; and
- removing the tubular element,
- wherein either the proximal implant part and the distal implant part are connected by the chord in the system, or the method comprises the additional step of connecting the proximal and distal implant parts by the chord.

Specifically, the present invention can provide a minimally invasive treatment or repairing an atrioventricular heart valve. The methods comprises in particular a transcatheter valve repairing method. The present invention is thereby compatible with and directed to different access points to a (beating) heart, such as transapical and in particular also a transfemoral approach. Usually valve replacement requires an open heart procedure with a "sternotomy", in which the chest is surgically separated for the procedure. Transcatheter procedures can be done through very small openings that leave all the chest bones in place. Therefore a transcatheter procedure provides beneficial treatment options to people who are considered an intermediate or high risk patient for standard valve replacement surgery while also providing the added bonus of a faster recovery in most cases.

### BRIEF DESCRIPTION OF THE DRAWINGS

Hereinafter, principles and embodiments of the invention are described referring to drawings. Same reference numbers in the drawings refer to same or analogous elements. The drawings show:
Fig.1: A schematic drawing of an instrument with a grabbing structure comprising a main body, a jaw and a counter portion.
Fig. 2: A schematic drawing of the instrument of Figure 1, wherein the counter portion has been moved in axial direction towards the jaw.
Fig. 3: A schematic drawing of the instrument of Figure 1, wherein the needle has been moved in axial direction through the jaw.
Fig.4: A schematic drawing of an instrument according to the invention with a grabbing structure comprising a main body and a jaw.
Fig. 5: A schematic drawing of the instrument according to Fig. 4, wherein the view is changed.
Fig. 6: A schematic drawing of the instrument according to Fig. 4 in a closed configuration.
Fig. 7: A schematic drawing of the instrument according to Fig. 4, wherein the needle reaches through the jaw.
Figs. 8A-8C: Schematic drawings of the instrument of Figure 4 in open configuration in a perspective view with different orientations.
Figs. 9A-9C: schematic drawings of an instrument according to the invention, wherein the first and second abutment surfaces define a plane being oblique to the tube axis.
Figs. 10A and 10B: Two representations of a grabbing structure comprising support wires and lateral grooves for the attachment of marker stripes.
Fig. 11: A schematic drawing of a main body being part of an instrument according to the invention showing a lateral groove for a marker.
Fig. 12: A schematic drawing of a jaw being part of an instrument according to the invention showing a lateral groove for a marker.
Figs. 13 and 14: A grabbing structure with antennae, in an open and a closed configuration; ¨
Figs. 15-17: A main body of an alternative grabbing structure and the grabbing structure in two different states, respectively;
Figs. 18 and 19: Variants of the grabbing structure of Figs. 15-17 with markers; and
Figs. 20 and 21: A grabbing structure with wing-like fold-out structure, in a folded-in and a folded-out configuration, respectively.

### DESCRIPTION OF THE PREFERRED EMBODIMENTS

The following more detailed description of the embodiments of the instrument is a representative of exemplary embodiments of the technology, wherein similar parts are designated by same numerals throughout. Standard medical planes of reference and descriptive terminology are employed in this specification. In particular, proximal means toward the trunk, or, in the case of an inanimate object, toward a user and distal means away from the trunk, or, in the case of an inanimate object, away from a user.

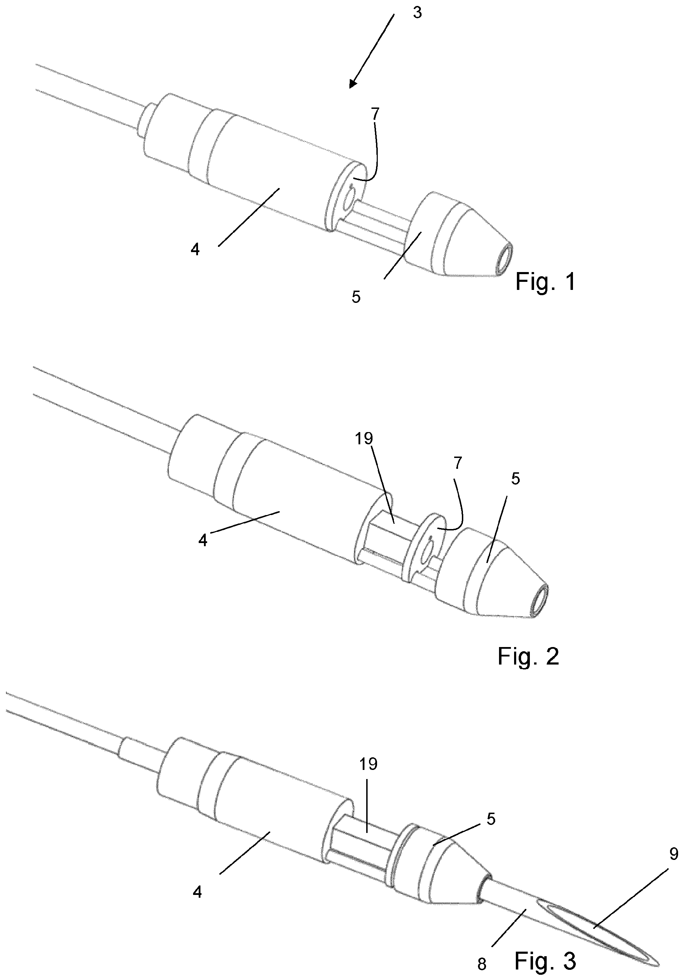

**Figures 1 to** 3 show one exemplary embodiment of a grabbing structure being part of an instrument for repairing an atrioventricular heart valve. Figure 1 shows a grabbing structure 3 comprising a main body 4 and a jaw 5 in an open position. The grabbing structure may be formed as a bullet tip grasper (bullet tip grabber). The grabbing structure may have a length of 0.75 to 1.2 cm. The jaw may be formed as a distal nosecone tip able to be advanced over two guiding rails to create a grasping opening. As shown in figure 2, the main body comprises a second abutment surface 7 formed by a press pad at the distal end of a counter portion 19 that is releaseable from the main body as shown in Fig. 2. The press pad is movable in axial direction towards the jaw to grasp and hold a leaflet. As can be seen in Figure 3 a cannulated needle 8 forming an inner tube 9 can be advanced from a proximal end of the grabbing structure (within a first tube which is not shown in Figures 1 - 3) through the main body, the counter portion and a channel within the jaw to protrude out of the grabbing structure. When the needle is advanced, it is able to puncture the grabbed leaflet. Within that needle, an implant may be arranged so that at least one part of the implant (distal implant part) may be implanted distally of the leaflet. The counter portion may be formed having a base being tube shaped or being elongated with an angular cross section and a channel for the cannulated needle. The press pad at the distal end of the counter portion may be round with at least one cut out to be sliceable on the guiding rails.

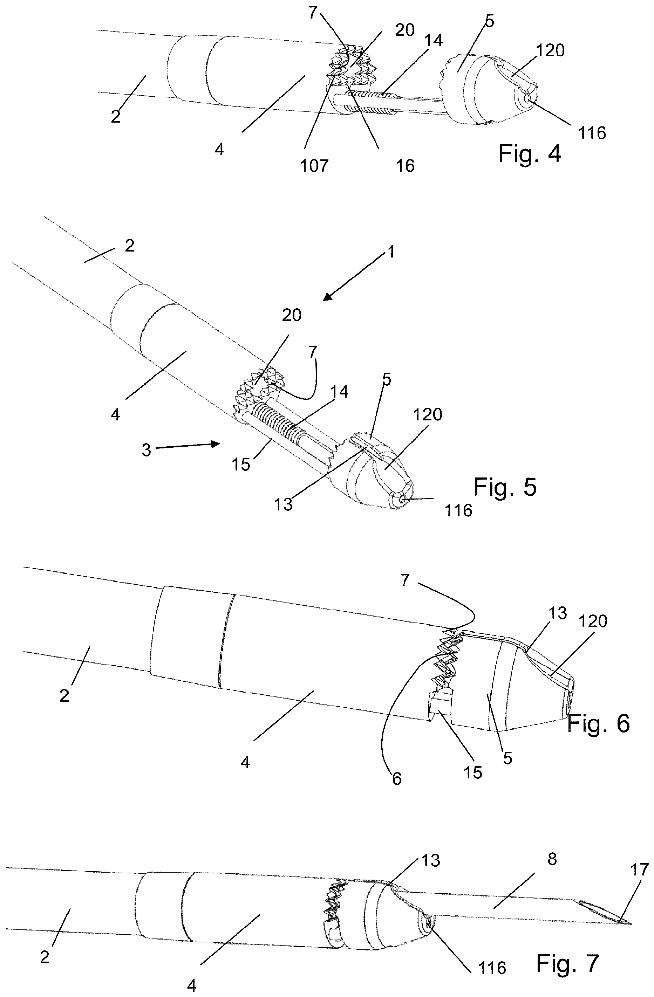

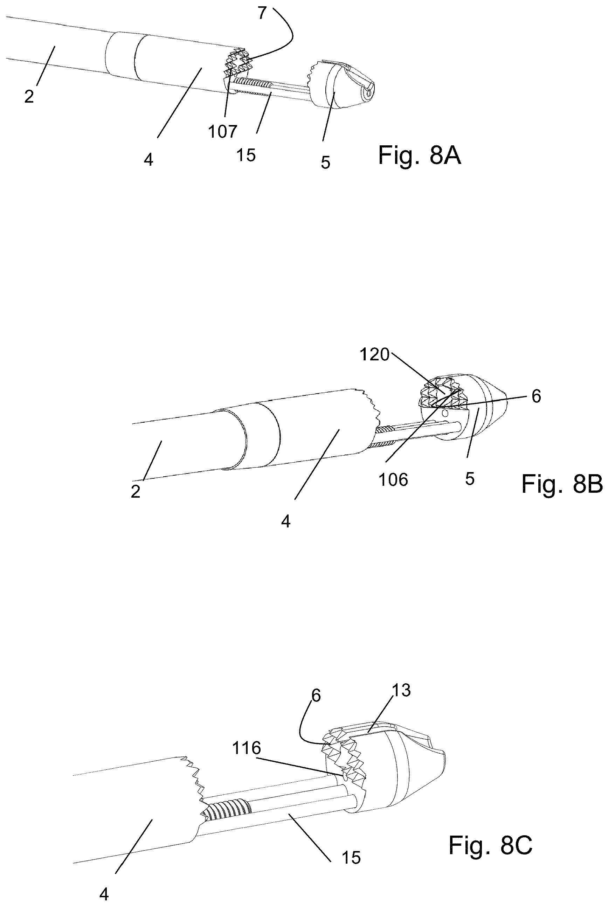

**Figures 4 to 8** **and** **8A-8C** show an exemplary embodiment of an instrument 1 according to the present invention. The instrument is shown in different perspective views. The proximal end of the main body 4 is attached to a first tube 2. The grabbing structure 3, the needle 8 and the implant can be manipulated via the first tube. This tube may be an implant catheter. For accessing the heart, the instrument may comprise a further, outer catheter (for example a transseptal and guide catheter) that is for example capable of being advanced through a suitable vein into the right atrium and from there into the left atrium, and from which the main body attached to the first tube 2 is deployed further into the left atrium to grab, together with the jaw, the leaflet. The tube may be a braided sheath with flexible section at the distal tip and a maneuvering section at the proximal end. For example, it may be a Pebax^{®} Catheter including a distal flexible section provides appropriate column strength and stiffness. The first tube can provide depth translation through and with the transseptal and guide catheter providing trajectory alignment to the heart valve, such as a mitral valve. The first tube may further comprise at least one marker band, such as a platinum band.

In contrast to the structure shown in Figures 1-3, in the embodiment of Figs. 4-8, 8A-8C and the embodiments described hereinafter, there is no separate counter portion capable of being advanced relative to the main body, but the main body 4 itself serves as the counter portion by comprising the distally facing second abutment surface 7.

The main body 4 can comprise a channel 20 for the cannulated needle 8 and a (central) guide wire channel 16 for a guide wire. The guide wire channel continues within the jaw 5 (see Figure 8 C and D; jaw guide wire channel portion 116 aligned with main body guide wire channel portion 16). The channel 20 for the cannulated needle is also continued within the jaw by the jaw portion 120 of the channel. This channel allows that the cannulated needle is advanced through the main body and the jaw and punctures the leaflet when held between the first and second abutment surfaces, and that one part of the implant can be released and implanted within a ventricle tissue (see hereinafter).

In the embodiment of Figs. 4-8, 8A-8C, the channel, more specifically the jaw portion 120 of the channel, has a slit-like lateral opening 13. After implantation of at least the distal part of the implant this opening allows to release an artificial chord attached to the implant parts carried by the cannulated needle. After withdrawal of the needle, the artificial chord can be released from the grabbing structure by a lateral movement.

The grabbing structure includes a mechanism to move the jaw in relation to the main body in axial directions. The mechanism comprises an operating structure such as an operating rod any may further comprise one or more guiding rods 15 (guiding rails).

In many embodiments, the operating structure is an operating rod (which can have a dimension to be bendable and therefore may be viewed as operating wire also) which is subject to axial movements from outside directly by the operator. Alternatively, as shown here, the movement may be facilitated by a threaded rod 14.In this alternative mechanism, the operator causes an axial movement by causing a rotation of the threaded rod. The embodiment with a threaded rod has the advantage of allowing a more precise determination of the axial position without the necessity of a feedback by means of monitoring but has the disadvantage that it may be sometimes difficult to transfer a rotational (torsional) movement across a comparably long distance of for example up to 1 m through the tubing..

Generally, while for illustration purposes most figures show an operating structure in the form of a threaded rod, a direct axial movement by an operating rod (operating wire) as well as an operating structure in the form of a threaded rod or other operating structures are options for all embodiments of the present invention.

The length of the grabbing structure in the open configuration may be 25 - 40 mm. Thereby the opening width is 8 to 15 mm, preferably 12 mm, the length of the main body is 10 - 20 mm and the jaw may have a length of 4 - 10 mm. The diameter of the grabbing structure may between 4.5 and 8 mm.

Figure 6 shows the grabbing structure in a closed or nearly closed configuration. Between the second abutment surface 7 of the main body 4 and the first abutment surface 6 of the jaw 5 a leaflet can be clamped and held.

Figure 7 shows the grabbing structure in a closed or nearly closed configuration and with a cannulated needle 8 advanced to protrude distally from the jaw 5. The cannulated needle has a sharpened distal end 17.

The first abutment surface 6 and the second abutment surface 7 define a plane. This plane and the tube axis form an angle of 90° in the embodiment shown in Figs.4-8, 8A-8C. In alternative embodiments, see also the embodiments described hereinafter, the angle may also be smaller.

As can be seen in Figures 8 A and B as well as in other figures, the first abutment surface 6 and/or second abutment surface 7 may be structured by having a surface structure 106, 107 forming a pattern of protrusions/indentations. Exemplarily, small pyramids having a square base are shown as the surface structures 106, 107. The surface structures 106, 107 may be formed to be complementary. This means that the structures fit to each other in case the grabbing structure is in a closed configuration. Therefore, the structures allow to increase the grip but in closed configuration there exists no risk for unwanted clamping of other structures than the leaflet because no open spaces are left between the main body 4 and the jaw 5.

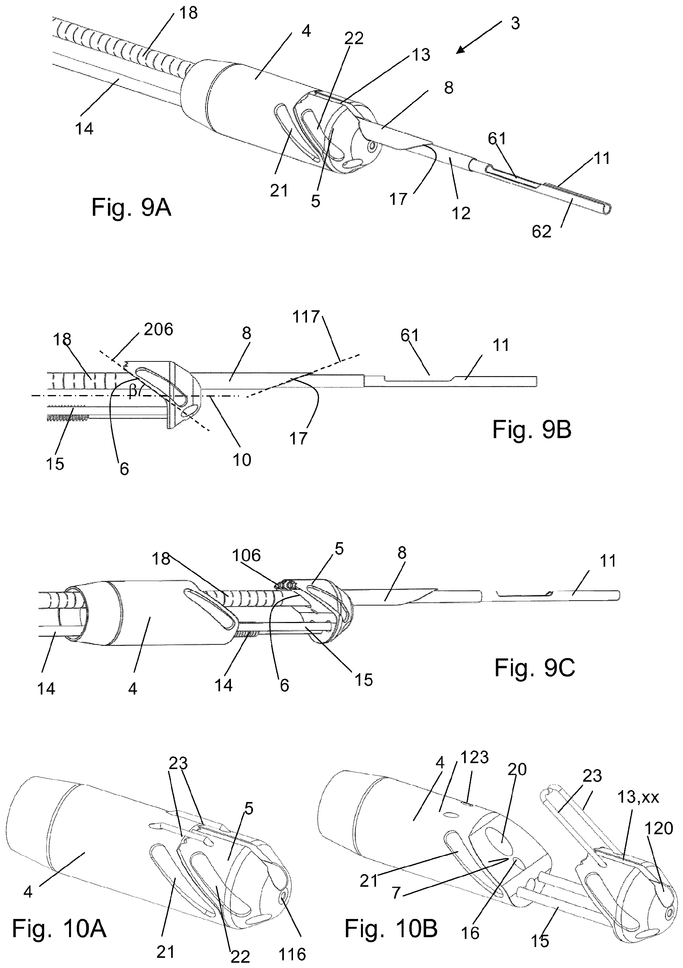

**Figures 9A-9C** show another embodiment of a grabbing structure 3 being part of an instrument 1 according to the present invention.

A first difference between the hereinbefore described embodiments and the embodiment of Figs. 9A-9C is that the abutment surfaces 6, 7 - which are parallel to each other - are not at a right angle to the axis 10 but have at least a section that is not perpendicular thereto. Especially, in the embodiment of Figs. 9A-9C, the abutment surfaces are not perpendicular to the axis at the position where the channel for the cannulated needle 8 goes through them.

In the specific depicted embodiment, the first abutment surface and the second abutment surface comprise each three portions. In closed position (Figure 9A) the corresponding portions of these abutment surfaces thus define three planes. Two planes form with the tube axis an angle of 90° or nearly 90°. The plane defined by a middle portion connecting the perpendicular portion forms with the tube axis an angle β of for example between 25 and 80°, for example between 30° and 60°. The cannulated needle 8 is pushed through the abutment surfaces within this section defining the plane being oblique in relation to the tube axis.

Other shapes of the abutment surfaces other than perpendicular are possible, including locally curved surfaces.

One possible feature of embodiments having abutment surfaces that are not perpendicular to the axis is also illustrated in Fig. 9B. The plane 206 defined by the abutment surfaces in vicinity of the channel for the cannulated needle is non-parallel to the plane 117 defined by the distal end 17 of the needle 8. Thereby, when the needle pierces the grabbed leaflet, the distal needle end does not lie flat against the leaflet, but for example the tip pierces the leaflet first.

Especially, the middle plane 117 defined by the distal end may be inclined, with respect to the axis, in a direction different from the direction of inclination of the plane 206 defined by the abutment surfaces around the channel (being a middle plane of the mouth of the channel in the abutment surfaces) and for example inclined in approximately the opposed direction, as illustrated in the shown embodiment.

The cannulated needle 8 may comprise a laser cut section 18 proximal of the sharpened distal end. Such section comprises a plurality of cuts in the otherwise cylindrical body, increases the flexibility of the needle and allows to implant one implant part within a ventricle tissue such as the ventricle wall or a papillary muscle. The laser cuts run on the circumference of the needle in form of an open ring. Thus, the cuts may have the form of circle arcs. It is preferred that the laser cuts in form of circle arcs are arranged alternating, so that the not cut part on each circle varies in position.

The cannulated needle 8 is shown to be in advanced position so that the distal end of the cannulated needle protrudes over the distal end of the jaw 5. Anchor carrier or implant carrier 11 is located within the needle and surrounded by an optional implant sleeve 12 or alternatively directly by the needle. The implant sleeve 12 and the implant carrier may be advanced to protrude distally from the needle. The implant carrier is designed to accommodate the implant parts. After the implant sleeve is withdrawn, the implant may be released. he implant carrier may have a lateral seat 61 at least for the proximal implant part. A portion distally thereof serves as foot portion 62 for preventing the proximal implant part from slipping out of the implant sleeve 12 as long as the proximal implant part is within the implant sleeve 12 (or, if there is no implant sleeve 12 within at least the cannulated needle 8).

The implant sleeve and the implant carrier may be made from nitinol and may also comprise a laser cut section which may be formed in the same manner as the laser cut section of the cannulated needle.

An even further feature of the embodiment of Figs. 9A-9C - which may independently of the other features described referring to these Figs be implemented in any embodiment - refers to markings. The main body 4 comprises a groove 21 for a marker stripe and the jaw 5 a groove 22 for another marker stripe. The grooves may be located near the edge of the abutment surfaces and may be curved. In these grooves a stripe of a radiopaque material or of a material yielding a good contrast in echography (sonography) can be attached. For example stripes of a platinum-iridium alloy can stick in these grooves. Such strips look under X-radiation like two hooks and allow to determine the orientation of the grabbing structure as well as if and to what extent the grabbing structure is open.

**Figures 10A and 10B** each show a representation of another embodiment of a grabbing structure 3 being part of an instrument according to the present invention. Fig. 10A shows the grabbing structure in closed form, and Fig. 10B depicts the same in an open position.

The grabbing structure in addition to the features described hereinbefore comprises further a fold-out support, such as a wire support 23. A fold-out support or other fold-out structure is equipped to fold out and to protrude radially-outward out of the cylindrical volume defined by the outer surface of the main body and jaw, and for example out of the outer catheter (such as transseptal guide catheter). The fold-out structure may for example fold out automatically upon release from such outer catheter, upon the grabbing structure being opened and/or upon a trigger action induced by the operator.

A fold-out support enhances the effective area for cooperating with the leaflet and hence may facilitate grasping the leaflet. It may be folded out from the jaw or from the main body. It is also possible to combine these two options. Especially, to be able to support the leaflet, the wires may increase the area of at least one abutment surface.

In the depicted embodiment, the fold-out support is a wire support consisting for example of two wires. In the open position the wires support the grasped leaflet, and thus promote the grasping of the leaflet. The wire support can consist of one wire loop or of two wires attached in parallel. Such wires may be bent at the free end. A wire support of two wires is preferably arranged to comprise a gap between the two wires which is aligned with the recess 13 of the channel 120 so as to allow releasing the chord from the needle arrangement.

The wire support can be arranged in a way that it is closely attached to the grabbing structure in the closed position but can stick out in the open position. Therefore the free ends of the wires attached to the jaw may be located in support recesses 123 within the main body. While the jaw moves away from the main body, the wires are pulled out and unfold or flip open. The wire supports may be made of a material having shape memory effect such as nitinol. Alternatively the wires may be made of a material being radiopaque or contain radiopaque marker.

A further feature of the embodiment of Figs. 10A and 10B as well as of embodiments described hereinafter - which further feature may be implemented independent of the other features of this embodiment - is the absence of a threaded rod for causing the axial movement of the jaw relative to the main body. Instead, one or both of the rods 15 that serve as guiding rods may be connected to a push-pull operating mechanism (essentially a push-pull wire) and thereby also serve for operating.

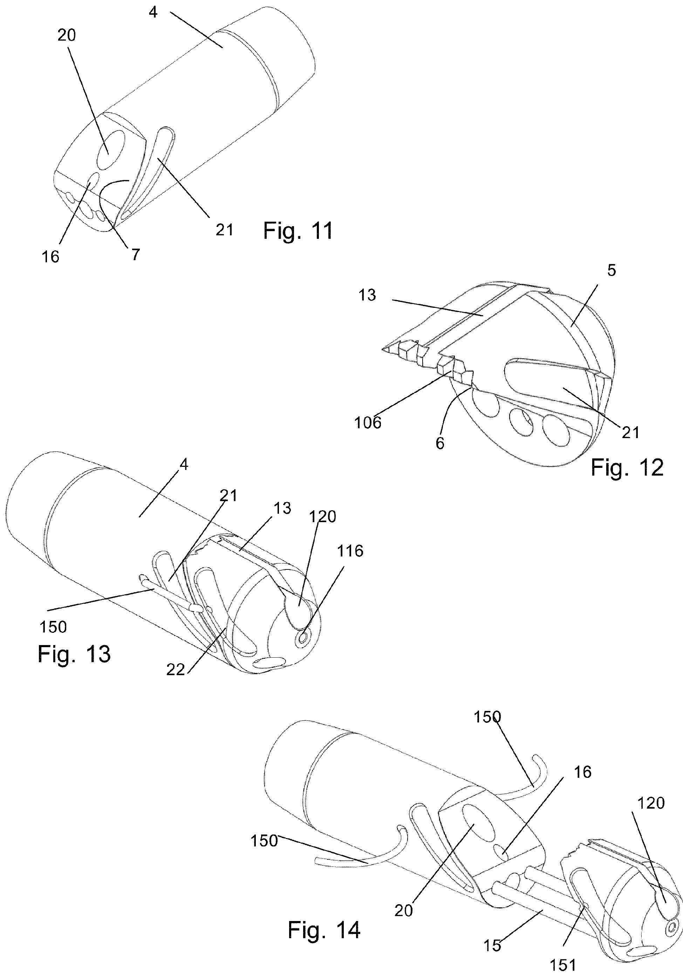

**Figure 11** shows a drawing of a main body 4 of a grabbing structure comprising a channel 16 for a guide wire and the channel 20 for a cannulated needle. Besides the abutment surface 7 the main body comprises a groove 21. Within said groove 21 a radiopaque marker can be bond. There may be an analogous groove on the other side of the main body. The groove can be 3 to 8 mm long, 0.15 to 0.5 mm deep and has a width between 1.5 and 4 mm.

**Figure** 12 shows a drawing of a jaw 5 of a grabbing structure, the jaw being equipped for cooperating with a main body as shown in Fig. 11. The jaw comprises a channel for a cannulated needle, the channel having a recess 13 so that the channel is open to the top. Besides the abutment surface, the jaw comprises a groove 22. Within said groove 21 a radiopaque marker can be bond. There may be an analogous groove on the other side of the jaw. The groove can be 3 to 8 mm long, 0.15 to 0.5 mm deep and has a width between 1.5 and 4 mm.

**Figures 13 and 14** show a variant of the embodiments of Figures 9-12 in the closed condition and in the open condition of the grabbing structure, respectively. In contrast to the previously described embodiments, the grabbing structure comprises a fold-out structure in the form of at least one fold-out antenna. In the embodiment of Figs. 13 and 14, it comprises two fold-out antennae, both attached laterally to the main body. The antennae increase, by radially protruding away from the main body and/or the jaw in the unfolded condition, the visibility when echography is applied. Much like a wire support of the above-described kind, such antenna can be arranged in a way that it is closely attached to the grabbing structure in the closed position but can stick out in the open position. Also the free end of the antenna may be seated in an according recess, such as an antenna recess 151 in the jaw. When the grabbing structure is deployed from the outer catheter or when the jaw moves away from the main body, the antenna is folded out. Also the antenna may be made of a material having shape memory effect such as nitinol and/or of a material being radiopaque or contain radiopaque marker.

In the embodiments of Figures 4-14 described hereinbefore, the channel for the cannulated needle is open towards a single lateral side by there being formed a slit-shaped recess in the jaw, whereas the - proximal - main body does not have such feature. For releasing the proximal implant part, the proximal implant part - for example carried by the anchor carrier as shown in Figs. 9A-9C - has to be deployed from the main body. If the proximal implant part is to lie proximally of the leaflet, deployment, therefore, may be done after the leaflet has been released from the grabbing structure.

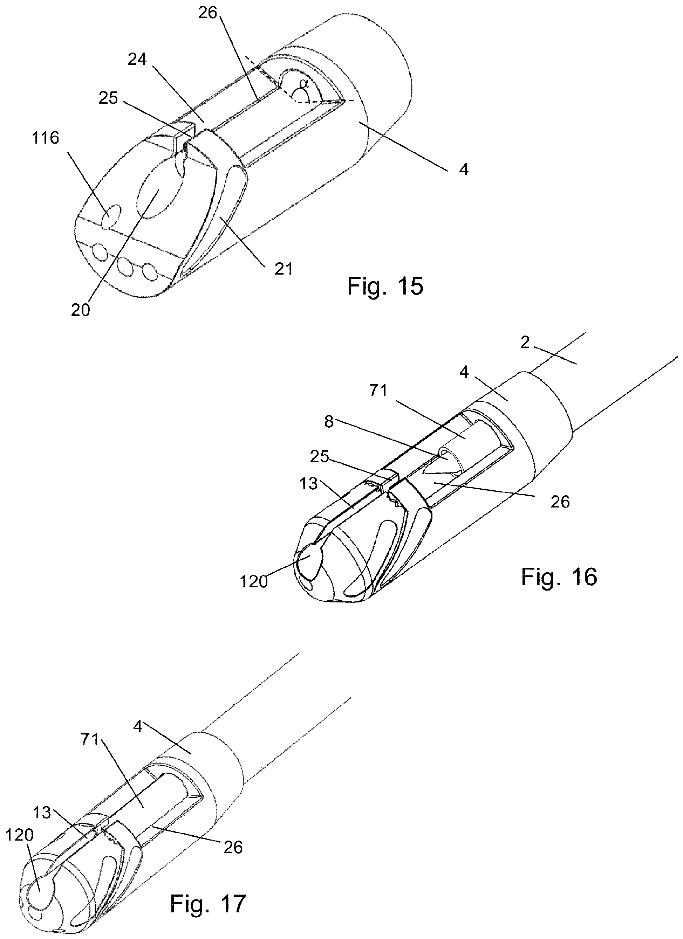

The embodiments of **Figures 15-19** described hereinafter are equipped for the option of there being a proximal anchor release while the leaflet is still being grabbed. This makes additional control by the operator possible - i.e. both is possible: release of the proximal implant part while the leaflet is still being grabbed, or release of the proximal implant part after release of the leaflet.

To this end, not only the channel portion 120 in the jaw but also the main body channel portion 20 is open to a lateral side (i.e. the same side as the jaw channel portion) by the main body 4 having a recess 24 extending from the distal end. In the depicted embodiment, the recess has a narrow distal recess portion 25 and a broader proximal recess portion 26 serving as implant release portion. The length (axial extension) and width of the recess or, if present, of its implant release portion 26 are sufficient for the proximal implant part to be released therethrough. Especially, the opening angle α of the main body recess portion or of its implant release portion may be at least 45° or at least 60° or at least 80° or more. The length is larger than the length of the proximal implant portion and hence larger than the length of the anchor carrier seat 61.

In Figures 16 and 17 a further optional feature is illustrated. Namely, the cannulated needle 8 is encompassed by a needle guide tube 71. The needle guide tube encompasses the needle 8 essentially up to its distal end during the forward (towards distally) movement of the needle for piercing the leaflet while the leaflet is being clamped. The needle guide tube 71 is retracted thereafter for release of the proximal implant part. The needle guide tube 71 makes sure that the needle is guided towards and into the jaw channel portion even if the leaflet causes a mechanical resistance against the forward movement/puncturing and even though the proximal recess portion is relatively wide open.

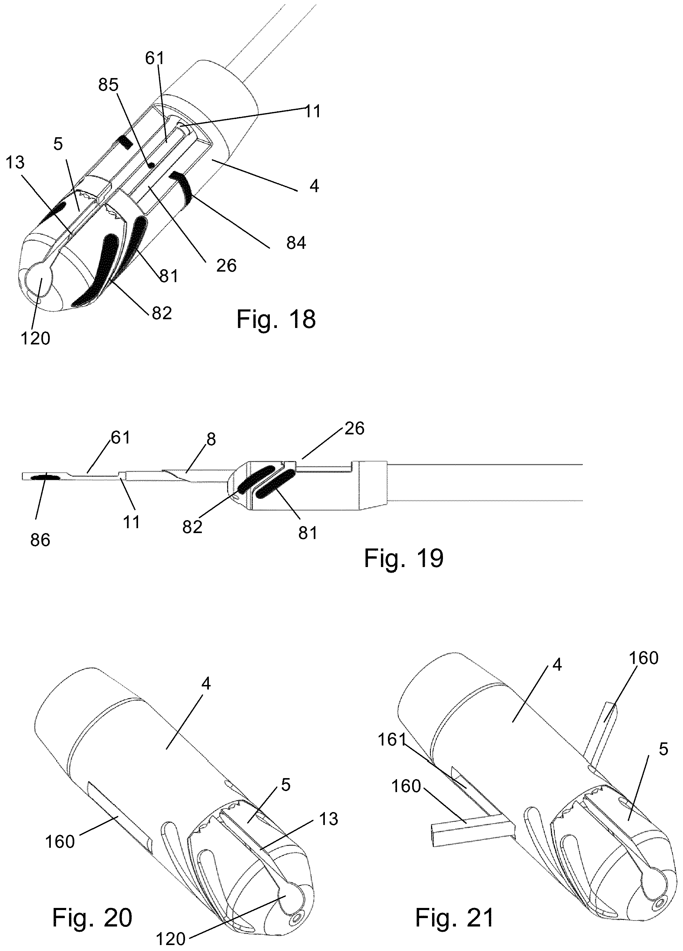

Figures 18 and 19 illustrate possible positions of markings that may be used to make sure that the axial positions of the anchor carrier 11 and of the implant release portion 26 are properly adjusted for implant release even if the device is too flexible for the operator to be able to define the relative positions precisely enough for the deployment of the proximal anchor just from markings on the handle device. In Fig. 84, the main body 4 comprises a circumferential (extending by 280° in the depicted embodiment) marker 84 the axial position of which defines an axial position of a central anchor carrier marker 85. According to a second option, a distal anchor carrier marker 86 may be aligned with the side markers 81, 82 of the main body 4 and/or the jaw 5, which are present in the marker grooves 21, 22 described hereinbefore.

The embodiment of **Figures 20 and 21** comprises a fold-out structure that is constituted by wing-like elements 160 capable of being folded out from the main body to assist orienting the grabbing structure with respect to the leaflet, especially by being visible by echography and/or radiography. Depending on the position and structure, a wing-like element may, in embodiments different from the one shown in Figs. 20 and 21, also act to enhance the effective area for supporting the leaflet and thereby also work as fold-out support. In the folded-in condition (Figure 20), the wing-like elements 160 are accommodated in a seat 161 so as to not protrude radially-outward out of the cylindrical volume defined by the outer surface of the main body and jaw, whereas in the folded-out condition (Figure 21) they radially protrude outward of this cylindrical volume. The fold-out process may take place automatically upon the grabbing structure being released from the outer tube it is guided in.

## Claims

1. An instrument for repairing an atrioventricular heart valve in a minimally invasive manner, the instrument comprising:
a tubular arrangement defining an axis (10) and an axial direction;
a leaflet grabbing structure (3), the leaflet grabbing structure (3) comprising a main body (4) and a jaw (5);
wherein the jaw (5) comprises a first proximally facing abutment surface (6) and the main body (4) comprises a second, distally facing abutment surface (7);
the instrument further comprising a needle (8), wherein the needle is cannulated and forms an inner tube, and wherein the needle (8) is movable relative to the main body (4) in axial directions,
whereby the leaflet grabbing structure is capable of clamping a leaflet of the heart valve between the first and second abutment surfaces (6, 7);
wherein the main body (4) and the jaw (5) have a channel (20, 120) being formed to allow the needle (8) to extend there through while the leaflet is being clamped;
**characterized in that** the channel (20, 120) is open to a lateral side by at least the jaw (5) comprising a lateral recess or slit (13; 25, 26).

2. The instrument according to claim 1, wherein the channel (20, 120) has a position that is not central with respect to the axis (10).

3. The instrument according to claim 1 or 2, wherein the jaw (5) is movable relative to the main body (4) in the axial direction.

4. The instrument according to any one of claims 1 - 3, wherein the instrument further comprises an anchor carrier (11) being arranged within the inner tube and being axially movable relative to it, the anchor carrier being configured to carry at least a part of an implant that is secured or capable of being secured to an artificial chord, wherein the instrument is preferably configured for a distal implant part and a proximal implant part being arranged beside one another, with the proximal implant part arranged proximally of the distal implant part, wherein the anchor carrier forms an anchor seat (61) and is configured to carry the proximal implant part of the implant, and wherein preferably the inner tube or a sleeve element (12) inside the inner tube prevents the proximal implant part from escaping from the anchor carrier (11) as long as the proximal implant part is received in the anchor seat (61) and is within the inner tube or sleeve element (12).

5. The instrument according to any one of claims 4, wherein the anchor carrier (11) comprises a marker (86) for determining a position by echography and/or radiography, and wherein the main body (4) and/or the jaw (5) comprises a marker (81, 82) for determining a position by echography and/or radiography, for determination of relative positions of the anchor carrier (11) and of the main body (4) and/or jaw (5).

6. The instrument according to any one of the previous claims, wherein the channel (20, 120) has a main body channel (20) and a jaw channel (120) aligned with the main body channel (20), wherein the jaw channel (120) is open to the lateral side along its full axial length, and wherein also a distal portion of the main body channel (120) is open to the lateral side.

7. The instrument according to claim 6, wherein the channel (20, 120) is open to the lateral side by a recess (13, 25, 26) that has a distal recess portion (13, 25) and proximally thereof, an implant release portion (26), wherein the implant release portion (26) of the recess runs in the main body (4), wherein the implant release portion (26) of the recess is wider than the distal recess portion (13, 25), and wherein preferably the distal recess portion (13, 25) has a first sub-portion (13) running in the jaw (5) and a second sub-portion (25) running in the main body (4), distally of the implant release portion (26).

8. The instrument according to claim 7, wherein an opening angle α of the implant release portion (26), by which the channel is open to the lateral side, is at least 45°.

9. The instrument according to any one of the previous claims, further comprising a needle guide (71) being a tube encompassing the cannulated needle (8) and being accommodated inside the channel (20, 120).

10. The instrument according to any one of the previous claims, wherein the first and second abutment surfaces (6, 7) at a position around a mouth of the channel (20, 120) in the first and second abutment surfaces (6, 7), define at least one plane being at an angle different from 90° to the axis (10), wherein a distal end of the needle (8) defines a distal needle end plane (117) that is at an angle different from 90° to the axis (10), and wherein the distal needle end plane (117) and the plane (206) defined by the first and second abutment surfaces (6, 7) are inclined in different directions.

11. The instrument according to any one of the previous claims, wherein the needle (8) has a sharpened distal end (17) and proximally therefrom a laser cut section (18).

12. The instrument according to any one of the previous claims, wherein the tubular arrangement comprises a first tube (2), wherein the leaflet grabbing structure is arranged at a distal part of the first tube and is contiguous to the first tube, wherein the needle is releasable from the first tube and movable relative to the main body (4) in axial directions, and/or wherein the tubular arrangement comprises an outer catheter being configured as a guide catheter, the outer catheter being dimensioned to accommodate the leaflet grabbing structure within an interior of the outer catheter.

13. The instrument according to any one of the previous claims wherein the jaw (5) and the main body (4) each comprise a marker (81, 82) for determining the relative positions of the jaw and the main body by echography and/or radiography.

14. The instrument according to any one of the previous claims, further comprising a fold-out structure (160) equipped to fold out and to protrude, when folded out, radially-outward out of the cylindrical volume defined by the outer surface of the main body and jaw, wherein the fold-out structure (160) is a fold-out support extending an area of at least one of the abutment surfaces.

15. A set, comprising an instrument for repairing an atrioventricular heart valve according to any one of the previous claims,
the set further comprising an implant that has a proximal implant part, a distal implant part, and a chord connecting or equipped to connect the proximal implant part and the distal implant part.

## Patentansprüche

1. Instrument zum Reparieren einer atrioventrikulären Herzklappe in einer minimal invasiven Weise, wobei das Instrument umfasst:
eine röhrenförmige Anordnung, die eine Achse (10) und eine axiale Richtung definiert;
eine Klappensegel-Greifstruktur (3), wobei die Klappensegel-Greifstruktur (3) einen Hauptkörper (4) und eine Klemmbacke (5) umfasst;
wobei die Klemmbacke (5) eine erste, proximal ausgerichtete Anlagefläche (6) umfasst und der Hauptkörper (4) eine zweite, distal ausgerichtete Anlagefläche (7) umfasst;
wobei das Instrument ferner eine Nadel (8) umfasst, wobei die Nadel kanüliert ist und ein Innenrohr bildet, und wobei die Nadel (8) relativ zu dem Hauptkörper (4) in axialen Richtungen beweglich ist,
wodurch die Klappensegel-Greifstruktur in der Lage ist, ein Klappensegel der Herzklappe zwischen der ersten und der zweiten Anlagefläche (6, 7) einzuklemmen;
wobei der Hauptkörper (4) und die Klemmbacke (5) einen Kanal (20, 120) aufweisen, der so ausgebildet ist, dass sich die Nadel (8) durch ihn hindurch erstrecken kann, während das Klappensegel eingeklemmt wird;
**dadurch gekennzeichnet, dass** der Kanal (20, 120) zu einer seitlichen Seite hin offen ist, indem zumindest die Klemmbacke (5) eine seitliche Aussparung oder einen Schlitz (13; 25, 26) aufweist.

2. Instrument nach Anspruch 1, wobei der Kanal (20, 120) eine Position hat, die nicht mittig zur Achse (10) liegt.

3. Instrument nach Anspruch 1 oder 2, bei dem die Backe (5) relativ zum Hauptkörper (4) in axialer Richtung beweglich ist.

4. Instrument nach einem der Ansprüche 1 bis 3, wobei das Instrument ferner einen Ankerträger (11) umfasst, der innerhalb des Innenrohrs angeordnet und relativ zu diesem axial beweglich ist, wobei der Ankerträger so konfiguriert ist, dass er zumindest einen Teil eines Implantats trägt, das an einer künstlichen Sehne befestigt ist oder befestigt werden kann, wobei das Instrument vorzugsweise so konfiguriert ist, dass ein distaler Implantatteil und ein proximaler Implantatteil nebeneinander angeordnet sind, wobei das Instrument vorzugsweise so ausgebildet ist, dass ein distales Implantatteil und ein proximales Implantatteil nebeneinander angeordnet sind, wobei das proximale Implantatteil proximal des distalen Implantatteils angeordnet ist, wobei der Ankerträger einen Verankerungssitz (61) ausbildet und zum Tragen des proximalen Implantatteils des Implantats ausgebildet ist, und wobei vorzugsweise das Innenrohr oder ein Hülsenelement (12) im Inneren des Innenrohrs ein Austreten des proximalen Implantatteils aus dem Ankerträger (11) verhindert, solange das proximale Implantatteil im Verankerungssitz (61) aufgenommen ist und sich innerhalb des Innenrohrs oder Hülsenelements (12) befindet.

5. Instrument nach einem der Ansprüche 4, wobei der Ankerträger (11) einen Marker (86) zur echographischen und/oder radiographischen Positionsbestimmung aufweist, und wobei der Hauptkörper (4) und/oder der Klemmbake (5) einen Marker (81, 82) zur echographischen und/oder radiographischen Positionsbestimmung aufweist, zur Bestimmung von Relativpositionen des Ankerträgers (11) und des Hauptkörpers (4) und/oder der Klemmbacke (5).

6. Instrument nach einem der vorhergehenden Ansprüche, wobei der Kanal (20, 120) einen Hauptkörperkanal (20) und einen mit dem Hauptkörperkanal (20) ausgerichteten Backenkanal (120) aufweist, wobei der Backenkanal (120) entlang seiner gesamten axialen Länge zur Seite hin offen ist und wobei auch ein distaler Abschnitt des Hauptkörperkanals (120) zur Seite hin offen ist.

7. Instrument nach Anspruch 6, wobei der Kanal (20, 120) zur lateralen Seite hin durch eine Ausnehmung (13, 25, 26) geöffnet ist, die einen distalen Ausnehmungsabschnitt (13, 25) und proximal dazu einen Implantatfreigabeabschnitt (26) aufweist, wobei der Implantatfreigabeabschnitt (26) der Ausnehmung im Hauptkörper (4) verläuft, wobei der Implantatfreigabeabschnitt (26) der Ausnehmung breiter ist als der distale Ausnehmungsabschnitt (13, 25), und wobei vorzugsweise der distale Ausnehmungsabschnitt (13, 25) einen ersten Teilabschnitt (13), der in der Klemmbacke (5) verläuft, und einen zweiten Teilabschnitt (25), der im Hauptkörper (4) verläuft, distal vom Implantatfreigabeabschnitt (26) aufweist.

8. Instrument nach Anspruch 7, wobei ein Öffnungswinkel α des Implantatfreigabeabschnitts (26), durch den der Kanal zur lateralen Seite hin offen ist, mindestens 45° beträgt.

9. Instrument nach einem der vorhergehenden Ansprüche umfassend ferner eine Nadelführung (71), die ein Rohr ist, das die kanülierte Nadel (8) umschliesst und im Kanal (20, 120) aufgenommen ist.

10. Instrument nach einem der vorhergehenden Ansprüche, wobei die ersten und zweiten Anlageflächen (6, 7) an einer Position um eine Mündung des Kanals (20, 120) in den ersten und zweiten Anlageflächen (6, 7) mindestens eine Ebene definieren, die in einem von 90° verschiedenen Winkel zur Achse (10) steht, wobei ein distales Ende der Nadel (8) eine distale Nadelendebene (117) definiert, die in einem von 90° verschiedenen Winkel zu der Achse (10) steht, und wobei die distale Nadelendebene (117) und die Ebene (206), die durch die erste und zweite Anlagefläche (6, 7) definiert ist, in verschiedene Richtungen geneigt sind.

11. Instrument nach einem der vorhergehenden Ansprüche, wobei die Nadel (8) ein geschärftes distales Ende (17) und proximal davon einen lasergeschnittenen Abschnitt (18) aufweist.

12. Instrument nach einem der vorhergehenden Ansprüche, wobei die röhrenförmige Anordnung eine erste Röhre (2) umfasst, wobei die Klappensegel-Greifstruktur an einem distalen Teil der ersten Röhre angeordnet ist und an die erste Röhre angrenzt, wobei die Nadel von der ersten Röhre lösbar und relativ zum Hauptkörper (4) in axialen Richtungen beweglich ist, und/oder wobei die röhrenförmige Anordnung einen Aussenkatheter umfasst, der als Führungskatheter konfiguriert ist, wobei der Aussenkatheter so dimensioniert ist, dass er die Klappensegel-Greifstruktur innerhalb eines Innenraums des Aussenkatheters aufnimmt.

13. Instrument nach einem der vorhergehenden Ansprüche, wobei die Klemmbacke (5) und der Hauptkörper (4) jeweils einen Marker (81, 82) zur Bestimmung der relativen Positionen der Klemmbacke und des Hauptkörpers durch Echographie und/oder Radiographie aufweisen.

14. Instrument nach einem der vorhergehenden Ansprüche, umfassend ferner eine ausklappbare Struktur (160), die so ausgestattet ist, dass sie ausklappbar ist und im ausgeklappten Zustand radial nach aussen aus dem zylindrischen Volumen herausragt, das durch die Aussenfläche des Hauptkörpers und der Klemmbacke definiert ist, wobei die ausklappbare Struktur (160) eine ausklappbare Stütze ist, die sich über einen Bereich von mindestens einer der Anlageflächen erstreckt.

15. Set, umfassend ein Instrument zur Reparatur eines atrioventrikulären Herzens nach einem der vorhergehenden Ansprüche,
wobei das Set ferner ein Implantat umfasst, das einen proximalen Implantatteil, einen distalen Implantatteil und eine Sehne aufweist, die den proximalen Implantatteil und den distalen Implantatteil verbindet oder dazu ausgestattet ist, sie zu verbinden.

## Revendications

1. Instrument pour réparer une valve cardiaque atrio-ventriculaire d'une manière peu invasive, l'instrument comprenant :
une disposition tubulaire définissant un axe (10) et une direction axiale ;
une structure de préhension de feuillets de valve (3), ladite structure de préhension de feuillets de valve (3) comprenant un corps principal (4) et une mâchoire de serrage (5) ;
ladite mâchoire de serrage (5) comprenant une première surface de butée orientée proximalement (6) et ledit corps principal (4) comprenant une seconde surface de butée orientée distalement (7) ;
dans lequel l'instrument comprend en outre une aiguille (8), dans lequel l'aiguille est canulée et forme un tube interne, et dans lequel l'aiguille (8) est mobile par rapport au corps principal (4) dans des directions axiales,
de sorte que la structure de préhension de feuillets de valve est capable de pincer un feuillet de valve de la valve cardiaque entre les premières et deuxièmes surfaces de butée (6, 7) ;
dans lequel le corps principal (4) et la mâchoire de serrage (5) comportent un canal (20, 120) formé de telle sorte que l'aiguille (8) peut s'étendre à travers celui-ci tout en serrant le feuillet de valve ;
**caractérisé en ce que** le canal (20, 120) est ouvert sur un côté latéral **en ce qu'**au moins la mâchoire de serrage (5) comporte un évidement ou une fente latérale (13 ; 25, 26).

2. L'instrument selon la revendication 1, dans lequel le canal (20, 120) a une position qui n'est pas centrale par rapport à l'axe (10).

3. L'instrument selon la revendication 1 ou 2, dans lequel la mâchoire de serrage (5) est mobile par rapport au corps principal (4) dans la direction axiale.

4. L'instrument selon l'une des revendications 1 à 3, dans lequel l'instrument comprend en outre un support d'ancrage (11) disposé à l'intérieur du tube intérieur et pouvant être déplacé axialement par rapport à celui-ci, le support d'ancrage étant configuré pour porter au moins une partie d'un implant fixé ou pouvant être fixé à une corde artificielle, dans lequel l'instrument est de préférence configuré pour une partie d'implant distale et une partie d'implant proximale disposées l'une à côté de l'autre, la partie d'implant proximale étant disposée proximalement par rapport à la partie d'implant distale, dans lequel le support d'ancrage forme un siège d'ancrage (61) et est configuré pour porter la partie proximale de l'implant, et le tube intérieur ou un élément de manchon (12) à l'intérieur du tube intérieur empêchant de préférence la partie proximale de l'implant de s'échapper du support d'ancrage (11) tant que la partie proximale de l'implant est reçue dans le siège d'ancrage (61) et se trouve à l'intérieur du tube intérieur ou de l'élément de manchon (12).

5. L'instrument selon l'une quelconque des revendications 4, dans lequel le support d'ancrage (11) comprend un marqueur (86) pour déterminer une position par échographie et/ou radiographie, et dans lequel le corps principal (4) et/ou la mâchoire (5) comprend un marqueur (81, 82) pour déterminer une position par échographie et/ou radiographie, pour la détermination des positions relatives du support d'ancrage (11) et du corps principal (4) et/ou de la mâchoire de serrage (5).

6. L'instrument selon l'une des revendications précédentes, dans lequel le canal (20, 120) a un canal de corps principal (20) et un canal de mâchoire (120) aligné avec le canal de corps principal (20), dans lequel le canal de mâchoire de serrage (120) est ouvert sur le côté latéral sur toute sa longueur axiale, et dans lequel également une partie distale du canal de corps principal (120) est ouverte sur le côté latéral.

7. L'instrument selon la revendication 6, dans lequel le canal (20, 120) est ouvert sur le côté latéral par un évidement (13, 25, 26) qui a une partie d'évidement distale (13, 25) et proximalement à celle-ci, une partie de libération de l'implant (26), dans lequel la partie de libération de l'implant (26) de l' évidement s'étend dans le corps principal (4), dans lequel la partie de libération de l'implant (26) de l'évidement est plus large que la partie d'évidement distale (13, 25), et dans lequel, de préférence, la partie d'évidement distale (13, 25) présente une première sous-portion (13) s'étendant dans la mâchoire de serrage (5) et une seconde sous-portion (25) s'étendant dans le corps principal (4), distalement par rapport à la partie de libération de l'implant (26).

8. L'instrument selon la revendication 7, dans lequel un angle d'ouverture α de la partie de libération de l'implant (26), par lequel le canal est ouvert sur le côté latéral, est d'au moins 45°.

9. L'instrument selon l'une quelconque des revendications précédentes, comprenant en outre un guide d'aiguille (71) qui est un tube englobant l'aiguille canulée (8) et qui est accommodé à l'intérieur du canal (20, 120).

10. L'instrument selon l'une quelconque des revendications précédentes, dans lequel les première et deuxième surfaces de butée (6, 7) à une position autour d'une embouchure du canal (20, 120) dans les première et deuxième surfaces de butée (6, 7), définissent au moins un plan formant un angle différent de 90° par rapport à l'axe (10), dans lequel une extrémité distale de l'aiguille (8) définit un plan d'extrémité distale de l'aiguille (117) qui forme un angle différent de 90° par rapport à l'axe (10), et dans lequel le plan d'extrémité distale de l'aiguille (117) et le plan (206) défini par la première et la deuxième surface de butée (6, 7) sont inclinés dans des directions différentes.

11. L'instrument selon l'une des revendications précédentes, dans lequel l'aiguille (8) a une extrémité distale aiguisée (17) et une section découpée au laser (18) dans sa partie proximale.

12. L'instrument selon l'une des revendications précédentes, dans lequel la disposition tubulaire comprend un premier tube (2), dans lequel la structure de préhension de feuillets de valve est disposée à une partie distale du premier tube et est contiguë au premier tube, dans lequel l'aiguille est libérable du premier tube et mobile par rapport au corps principal (4) dans des directions axiales, et/ou dans lequel la disposition tubulaire comprend un cathéter externe configuré comme un cathéter de guidage, le cathéter externe étant dimensionné pour accueillir la structure de préhension de feuillets de valve à l'intérieur du cathéter externe.

13. L'instrument selon l'une quelconque des revendications précédentes dans lequel la mâchoire de serrage (5) et le corps principal (4) comprennent chacun un marqueur (81, 82) pour déterminer les positions relatives de la mâchoire de serrage et du corps principal par échographie et/ou radiographie.

14. L'instrument selon l'une quelconque des revendications précédentes, comprenant en outre une structure dépliable (160) équipée pour se déplier et faire saillie, une fois dépliée, radialement vers l'extérieur du volume cylindrique défini par la surface extérieure du corps principal et de la mâchoire de serrage, la structure dépliable (160) étant un support dépliable s'étendant sur une zone d'au moins l'une des surfaces de butée.

15. Ensemble comprenant un instrument pour réparer une valve cardiaque valve cardiaque atrio-ventriculaire selon l'une des revendications précédentes,
l'ensemble comprenant en outre un implant qui a une partie d'implant proximale, une partie d'implant distale et une corde reliant ou équipée pour relier la partie d'implant proximale et la partie d'implant distale.
